# EUROPÄISCHE PATENTSCHRIFT

(11) **EP 2 934 473 B1**
(45) Veröffentlichungstag und Bekanntmachung des Hinweises auf die Patenterteilung: **04.04.2018**
(21) Anmeldenummer: 13811397.2
(22) Anmeldetag: 28.11.2013
(51) Int. Cl.: A61K 8/60, A61K 8/73, A61K 8/81, A61Q 19/00, A61K 8/20

(54) **REINIGUNGSZUBEREITUNGEN MIT PEELINGEFFEKT AUF DER BASIS VON WASSERLÖSLICHEN BESTANDTEILEN IN KRISTALLINER FORM**
CLEANSING PREPARATIONS WITH EXFOLIATING ACTION, BASED ON WATER-SOLUBLE CONSTITUENTS IN CRYSTALLINE FORM
COMPOSITIONS DE NETTOYAGE PRÉSENTANT UN EFFET PELAGE À BASE DE CONSTITUANTS SOLUBLES DANS L'EAU SOUS FORME CRISTALLINE

(30) Priorität: 19.12.2012 DE 102012223743
(43) Veröffentlichungstag der Anmeldung: 28.10.2015
(73) Patentinhaber: Beiersdorf AG, 20253 Hamburg (DE)
(72) Erfinder: SUKOWSKI, Verena, 20257 Hamburg (DE); ARGEMBEAUX, Horst, 21465 Wentorf (DE); RASCHKE, Thomas, 25421 Pinneberg (DE)
(86) Internationale Anmeldenummer: PCT/EP2013/074980
(87) Internationale Veröffentlichungsnummer: WO 2014/095296

(56) Entgegenhaltungen:
- EP-A2- 0 584 877
- WO-A1-2008/023145
- WO-A1-2008/034549
- WO-A1-2008/047148
- WO-A2-2012/167903
- US-A1- 2006 034 978

## Beschreibung

Die vorliegende Erfindung beschreibt kosmetische Reinigungszubereitungen, welche neben Tensiden wasserlösliche Bestandteile in gelöster und kristalliner Form enthalten, die durch polymere Strukturanten stabil in der Zubereitung verteilt sind. Die kristallinen Formen dieser wasserlöslichen Bestandteile, die als Peelingpartikel wirken, werden durch den Zusatz von Polyolen in ihrer Form und/oder Größe stabilisiert.

Die Reinigung des Körpers ist von alters her ein Bedürfnis des Menschen. In der Art und Weise und Häufigkeit der Reinigung hat es über die Jahrhunderte große Veränderungen gegeben. Heutzutage ist es für viele Menschen üblich, dass zumindest einmal am Tag eine Ganzkörperreinigung in Form eines Duschbades oder Vollbades erfolgt. Zu diesem Zweck verwandte Dusch-und Badezubereitungen sind an sich bekannt. Jedoch gibt es immer neue Wünsche und Bedürfnisse der Verbraucher, denen die kosmetische Industrie Rechnung trägt, indem immer wieder neue, verbesserte und veränderte Zubereitungen zur Verfügung gestellt werden.

Zur tiefer gehenden Hauterfrischung und -reinigung gibt es Reinigungsmittel, die einen Peeling-Effekt aufweisen.

Unter Peeling versteht man eine kosmetische oder dermatologische Behandlung, bei der die oberflächlichen Schichten der Haut abgetragen werden. Bei einem oberflächlichen Peeling wird die oberste Hornschicht der Haut mechanisch oder chemisch entfernt. Bekannt sind auch tiefer gehende Peelingverfahren, bei denen die gesamte Hornschicht oder auch die Haut bis zur Kollagenschicht abgetragen wird.

Unterschieden werden chemische und mechanische Verfahren. Beim chemischen Peeling werden Fruchtsäuren, Lipo-Hydroxy-Säure, Trichloressigsäuren, Phenolverbindungen und Vitamin A-Säure eingesetzt. Die beiden erstgenannten Verbindungsklassen sind für das oberflächliche Peeling geeignet.

Beim mechanischen Peeling können verschiedene Substanzen zum Einsatz kommen, von denen hier einige aufgeführt werden:
Aluminiumoxid-Mikropartikel, Polydimethylsilikonharz-Mikropartikel, harte Bürsten, Mikrofasertücher, Tonerde, Sand, Kunststoffpartikel, zerstoßene oder gemahlene Kerne von Aprikosen oder Pfirsichen, Wachs. Als besonders geeignet zur Einarbeitung in Reinigungszubereitungen für die Gesichtshaut haben sich Peelingpartikel aus Polyethylen erwiesen, die beispielsweise unter dem Handelsnamen Inducos 13-Series erhältlich sind. Der Nachteil dieser Peelingpartikel ist jedoch, dass sie synthetischen Ursprungs sind und der biologische Abbau dieser Partikel in Kläranlagen sehr langsam erfolgt, d.h. die Partikel belasten die Umwelt, insbesondere die Flüsse und die Meere.

Es war also wünschenswert, Peelingpartikel zur Verfügung zur stellen, die natürlichen Ursprungs sind, eine gute biologische Abbaubarkeit aufweisen und darüber hinaus eine vergleichbare Hautverträglichkeit und Wirkleistung wie die Peelingpartikel auf synthetischer Basis, insbesondere auf Polyethylenbasis haben. Es wurde gefunden, dass Zuckerkristalle eine gute Peelingwirkung zeigen und Haut-verträglich sind. Zudem lösen sich diese Kristalle bei der Anwendung, d.h. beim Abspülen mit Wasser weitgehend auf.

Im Stand der Technik gibt es bereits Dokumente, die die homogene Verteilung von Partikeln in Tensidsystemen beschreiben. Diese Systeme weisen jedoch einen charakteristischen Aufbau auf und werden im Englischen als "Structured Surfactant Systems" bezeichnet, im Deutschen wird im Folgenden die Bezeichnung strukturierte Tensidsysteme verwendet. Diese Systeme zeichnen sich durch eine lamellare Phase (auch Mesophase oder G-Phase genannt) und meist eine eingeschobene wässrige Phase aus. Die lamellare Phase ist durch Doppelschichten (im Englischen wird der Begriff Bilayer verwendet) von Tensiden gekennzeichnet, bei denen die hydrophoben Enden der Tenside in der Regel nach innen zeigen und die hydrophilen Enden nach außen. Die Doppelschichten liegen nebeneinander mit einer parallelen oder konzentrischen Ausrichtung, sie können durch wässrige Schichten getrennt sein. Eine besondere Eigenschaft dieser Systeme ist es, dass sie in der Lage sind, suspendierte Partikel in Lösung zu halten, aber auch ein Ausgießen oder Fließen zu erlauben.

Die meisten strukturierten Tensidsysteme enthalten Elektrolyte, Tenside und Wasser um feste Partikel in Schwebe zu halten.

Es gibt im Stand der Technik Dokumente, die strukturierte Tensidsysteme für den Einsatz in der Kosmetik beschreiben. Das Dokument EP 1203068 offenbart ein Tensidsystem mit erweiterten lamellaren Phasen, welches gebildet wird aus Tensiden, Wasser und Strukturanten, die ein wasserlösliches Kohlenhydrat oder auch ein Elektrolyt sein können. Ein derartiges Tensidsystem ist in der Lage Partikel stabil zu suspendieren.

Das Dokument WO 2008/023145 offenbart strukturierte Tensidsysteme, die zur Hautabschuppung verwendet werden. Die Hautabschuppung erfolgt durch feste Zuckerpartikel, die in eine gesättigte Lösung des Zuckers gegeben werden zusammen mit ausreichend Tensid. Als zusätzliche strukturgebende Substanzen werden Elektrolyte offenbart.

Die vorgenannten Schriften offenbaren strukturierte Tensidsysteme, die Partikel enthalten. In der Herstellung und im Zusammenspiel der einzelnen Komponenten sind strukturierte Systeme anspruchsvoll und der Umgang mit derartigen Systemen in der Kosmetik ist nicht frei von Rückschlägen.

Langzeituntersuchungen an Reinigungszubereitungen, die als Peelingpartikel Zuckerkristalle enthalten, zeigen das Phänomen, dass die Kristalle, insbesondere die Zuckerkristalle, im Laufe der Zeit immer größer werden. Es hat ein Kristallwachstum eingesetzt. Dies führt dazu, dass mit der Größenzunahme der Kristalle größere Peelingpartikel in der Zubereitung vorliegen, die nicht mehr den angenehmen Peelingeffekt der kleineren Kristalle haben. Dieses Phänomen führt zu drei Nachteilen; zum einen können die größeren Partikel, vor allem wenn sie intensiv auf der Haut hin und her gerieben werden, die Hautoberfläche reizen. Zum anderen ist die Sensorik der Zubereitung beeinträchtigt, es entsteht ein körniger Eindruck bei der Verwendung. Darüber hinaus lösen sich die größeren Kristalle nicht mehr so schnell auf beim Kontakt mit größeren Mengen Wasser während des Abspülvorganges.

Die Aufgabe, die der vorliegenden Erfindung zugrunde liegt, besteht zunächst darin, kosmetische Reinigungszubereitungen mit stabil suspendierten Partikeln, insbesondere Peelingpartikeln, zur Verfügung zu stellen, deren Zusammensetzung einfach zu formulierende Komponenten enthält und deren Herstellung auf einfache Weise erfolgt.

Weiterhin ist es Aufgabe der vorliegenden Erfindung, dass die Substanzen mit Peelingeffekt, insbesondere Kohlenhydratkristalle, homogen in der Zubereitung verteilt sind und auch bleiben.

Weiterhin ist es die Aufgabe der vorliegenden Erfindung, Zusammensetzungen zur Verfügung zu stellen, die einen Peelingeffekt durch einfache, natürliche Komponenten ermöglichen.

Ebenso ist es die Aufgabe der vorliegenden Erfindung, dass bei der Anwendung der erfindungsgemäßen Zubereitungen keine oder praktisch keine Rückstände auf Waschtischen oder Anwendungseinrichtungen hinterlassen werden.

Aufgabe der vorliegenden Erfindung ist es in einem weiteren Schritt, Zubereitungen mit Zuckerkristallen zur Verfügung zu stellen, die kein oder ein deutlich verlangsamtes Kristallwachstum aufweisen, so dass die Größe und Form der Kristalle konstant oder weitgehend konstant ist.

Aufgabe ist es auch, Reinigungszubereitungen mit Zuckerkristallen zur Verfügung zu stellen, deren Qualität und Reinigungsleistung über einen längeren Zeitraum stabil bleibt.

Weiterhin Aufgabe der vorliegenden Erfindung ist es, stabile Zubereitungen bereitzustellen. Stabil im Sinne der vorliegenden Erfindung bedeutet, dass es innerhalb der Zubereitung über einem längeren Zeitraum zu keiner nachweisbaren Phasentrennung kommt und sich keine nennenswerten Mengen an Partikeln, insbesondere Partikel aus Zuckerkristallen in der Zubereitung am Boden der Verpackungseinheit absetzen. Längerer Zeitraum bedeutet mehr als zwei Monate.

Darüber hinaus sollten Reinigungszubereitungen zur Verfügung gestellt werden, die bei reduzierter Zuckermenge vergleichbare Produktleistungen, beispielsweise Peelingwirkung, zeigen wie Zubereitungen mit einem höheren Gehalt an Zucker.

Die Aufgaben konnten für den Fachmann überraschend gelöst werden durch die Bereitstellung von kosmetischen Reinigungszubereitungen, die Tenside, wasserlösliche Bestandteile in gelöster und ungelöster Form und polymere Strukturanten enthalten. Die wasserlöslichen Bestandteile in ungelöster Form liegen ganz oder zumindest zum überwiegenden Teil in fester und/oder kristalliner Form vor.

Erfindungsgemäß sind Zubereitungen, die kein strukturiertes Tensidsystem enthalten.

Erfindungsgemäß ist, dass in den erfindungsgemäßen Zubereitungen anionische und/oder amphotere und/oder kationische und/oder nichtionische Tenside enthalten sind.

Der Gehalt an Tensiden in den erfindungsgemäßen Zubereitungen beträgt bevorzugt 1 bis 12 Gew.-%, besonders bevorzugt 2 bis 9 Gew.-%, bezogen auf das Gesamtgewicht der Zubereitung.

Die Tensidgehalte in den erfindungsgemäßen Zubereitungen können jedoch im Bereich von 0,1 bis 10 Gew.-% liegen. Dies sind Bereiche, in denen sich keine strukturierten Tensidsysteme ausbilden.

Erfindungsgemäß sind auch Zubereitungen mit einem Tensidgehalt > 10 bis 15 Gew.-%, bezogen auf das Gesamtgewicht der Zubereitung, die kein strukturiertes Tensidsystem aufweisen.

Erfindungsgemäß sind kosmetische Reinigungszubereitungen mit wasserlöslichen Bestandteilen in gelöster und fester und/oder kristalliner Form, wenn es sich bei diesen um Kohlehydrate handelt.

Die erfindungsgemäß einsetzbaren Kohlenhydrate können beispielsweise Saccharide sein, wie Mono-, Di-, Oligo- oder Polysaccharide. Mono- oder Disaccharide können beispielsweise Saccharose, Glukose oder Fruktose sein, wobei Saccharose bevorzugt ist. Es ist bevorzugt in den erfindungsgemäßen Zubereitungen jeweils ein Kohlenhydrat, insbesondere einen Zucker einzusetzen. Denkbar sind jedoch auch Mischungen.

Der Gesamtkohlenhydratgehalt beträgt 10 bis 90 % Gew.-%, bevorzugt 15 bis 80 Gew.-%, besonders bevorzugt 20 bis 70 Gew.-% bezogen auf das Gesamtgewicht der Zubereitung.

Es ist bevorzugt, dass in den erfindungsgemäßen Reinigungszubereitungen die polymeren Strukturanten vernetzte Copolymere sind, wobei Acrylat/C10-30 Acrylacrylat Crosspolymer und Copolymere aus Vinylpyrrolidon und Acrylsäure besonders bevorzugt sind.
Acrylat/C10-30 Acrylacrylat Crosspolymer und Copolymere aus Vinylpyrrolidon und Acrylsäure können in den erfindungsgemäßen Zubereitungen jeweils einzeln oder in Kombination zum Einsatz kommen. Die jeweiligen Ausführungsformen führen zu äußerst befriedigenden Reinigungszubereitungen.

Der Gehalt an polymeren Strukturanten beträgt 0,05 bis 3,0 %, bevorzugt 0,1 bis 2,0 Gew.-%, bezogen auf das Gesamtgewicht der Zubereitung.

Erfindungsgemäß vorteilhaft ist auch der Einsatz von Xanthangummi mit einem Gehalt von 0,05 bis 0,8 Gew.-%, bevorzugt 0,1 bis 0,5 Gew.-%.

Erfindungsgemäß sind ebenfalls kosmetische Reinigungszubereitungen mit
- Tensiden,
- Zuckern in gelöster und kristalliner Form und
- als Strukturanten vernetzte Copolymere aus Vinylpyrrolidon und Acrylsäure,
- aufweisend eine dynamische Viskosität < 80.000 mPa s bei 25°C,
- wobei die Verteilung der Zuckerkristalle in der Zubereitung homogen und stabil ist.

Erfindungsgemäß ist die Verwendung von polymeren Strukturanten zur Hemmung der Sedimentationsneigung von Kohlenhydratkristallen in Kohlenhydrat- und Tensid-haltigen kosmetischen Reinigungszubereitungen.

Erfindungsgemäß vorteilhaft ist die Verwendung von polymeren Strukturanten zur Hemmung der Sedimentationsneigung von Zuckerkristallen in Zucker-gesättigten, tensidhaltigen kosmetischen Reinigungszubereitungen.

Erfindungsgemäß ist auch ein Verfahren zur Hemmung der Sedimentationsneigung von Kohlenhydratkristallen in Kohlenhydrat-gesättigten, tensidhaltigen Reinigungszubereitungen dadurch gekennzeichnet, dass der Zubereitung polymere Strukturanten, bevorzugt vernetzte Copolymere aus Vinylpyrrolidon und Acrylsäure zugesetzt werden.

Darüber hinaus wurde überraschenderweise gefunden, dass die Einarbeitung von Polyolen, insbesondere Glycerin, in Reinigungszubereitungen enthaltend Zucker als wasserlöslichen Bestandteil, wobei ein Teil davon in Form von Zuckerkristallen vorliegt, das Kristallwachstum hemmt und die Kristallgröße weitgehend konstant bleibt, wobei der Gehalt der Polyole ≥ 15 Gew.-% ist.

Es sind zwar bereits Dokumente veröffentlicht, in denen der Einsatz von Polyolen, auch in Reinigungszubereitungen offenbart wird, z. B. EP 839522, EP 1529098. Aus keinem dieser Dokumente ist jedoch zu entnehmen, dass Polyole, insbesondere Glycerin, einen Einfluss auf das Kristallwachstum von Zuckerkristallen hat.

Erfindungsgemäß sind demnach Reinigungszubereitungen, die Tenside, Wasser und wasserlösliche Bestandteile in gelöster und kristalliner Form umfassen, wobei als wasserlösliche Bestandteile Zucker, von denen ein Teil in Form von Zuckerkristallen vorliegt, enthalten sind und wobei die Kristalle durch den Einsatz von polymeren Strukturanten homogen und stabil in der Zubereitung verteilt sind und durch Polyole mit einem Gehalt ≥ 15 Gew.-%, bezogen auf das Gesamtgewicht der Zubereitung, in ihrer Größe und Form stabilisiert werden.

Reinigungszubereitungen enthaltend Polyole mit einem Gehalt ≥ 15 Gew.-% werden im Folgenden als Zubereitungen enthaltend Kristall-stabilisierende Polyole bezeichnet.

Es ist bevorzugt, dass es sich in Zubereitungen enthaltend Kristall-stabilisierende Polyole bei den Zuckern um Mono- und/oder Di- und/oder Oligosaccharide handelt, wobei Saccharose insbesondere bevorzugt ist. Die Zucker sind mit einem Gehalt von 10 bis 90 Gew.-%, bevorzugt 15 bis 80 Gew.-%, insbesondere bevorzugt 20 bis 70 Gew.-%, bezogen auf das Gesamtgewicht der Zubereitung, in den Zubereitungen vorhanden sind.

Es ist bevorzugt, dass in den Zubereitungen enthaltend Kristall-stabilisierende Polyole die polymeren Strukturanten ausgewählt werden aus der Gruppe der Celluloseether, Polyacrylate und Polyacrylat Copolymere. Weiter bevorzugt sind Polyacrylat Copolymere, wobei Acrylic Acid/VP Crosspolymer, Acrylates Copolymer und Acrylates Crosspolymer-4 besonders bevorzugt sind. Ganz besonders bevorzugt ist das Acrylic Acid/VP Crosspolymer.

Der Gehalt an polymerem Strukturanten beträgt 0,05 bis 3,0 % Gew.-%, bevorzugt 0,1 bis 2,0 Gew.-% beträgt, bezogen auf das Gesamtgewicht der Zubereitung.

Es ist auch bevorzugt, dass die erfindungsgemäßen Zubereitungen Xanthangummi mit einem Gehalt vom 0,05 bis 0,8 Gew.-%, bevorzugt 0,1 bis 0,5 Gew.-% enthalten.

Es ist weiterhin bevorzugt, dass in den Zubereitungen enthaltend Kristall-stabilisierende Polyole die Tenside eine Kombination anionischer und nichtionische Tenside sind. Besonders bevorzugt sind anionische und/oder nichtionische Tenside, die eine Alkylgruppe mit einer Kettenlänge von 10-16 C-Atomen, insbesondere von 12-14 C-Atomen aufweisen. Es ist noch weiter bevorzugt, dass die anionischen und/oder nichtionischen Tenside Alkylgruppen aufweisen, die charakteristisch sind für Alkylgruppen, die aus der Aufarbeitung von Kokosöl zu Tensiden stammen.

Es ist ganz besonders bevorzugt, dass in den Zubereitungen enthaltend Kristall-stabilisierende Polyole die anionischen Tenside ausgewählt werden aus der Gruppe Alkylsulfate, Acylglutamate, Alkylsulfonate, Alkylsulfosuccinate und Acylsarcosinate sind, wobei Alkylsulfate besonders vorteilhaft sind.

Es ist auch besonders bevorzugt, dass in den Zubereitungen enthaltend Kristall-stabilisierende Polyole die nichtionischen Tenside Alkylglucoside sind.

Es ist ganz besonders bevorzugt, dass in den Zubereitungen enthaltend Kristall-stabilisierende Polyole die anionischen Tenside Alkylsulfate und die nichtionischen Tenside Alkylglucoside sind.

Der Tensidgehalt in den Zubereitungen enthaltend Kristall-stabilisierende Polyole beträgt 0,1 bis 10 Gew.-%, bevorzugt 0,5 bis 8,0 Gew.-%, bezogen auf den Aktivgehalt und das Gesamtgewicht der Zubereitung.

In den Zubereitungen enthaltend Kristall-stabilisierende Polyole werden die Polyole ausgewählt aus der Gruppe Glycerin und Propylenglycol. Es ist erfindungsgemäß, dass in den Reinigungszubereitungen Glycerin und Propylenglycol enthalten sind. Es ist bevorzugt, dass in den Reinigungszubereitungen als Polyol Glycerin enthalten ist. Der Gehalt an Polyolen beträgt 15 - 50 Gew.-%, bevorzugt 25 - 40 Gew.-%, bezogen auf das Gesamtgewicht der Zubereitung.

Erfindungsgemäß ist die Verwendung von polymeren Strukturanten zur Hemmung der Sedimentationsneigung von Kohlenhydratkristallen in Kohlenhydrat- und Tensid-haltigen kosmetischen Reinigungszubereitungen. Bevorzugt ist, dass die Reinigungszubereitungen Tenside, Wasser und wasserlösliche Bestandteile in gelöster und kristalliner Form umfassen, wobei als wasserlöslicher Bestandteile Zucker, von dem ein Teil in Form von Zuckerkristallen vorliegt, enthalten ist und wobei die Kristalle durch den Einsatz von polymeren Strukturanten homogen und stabil in der Zubereitung verteilt sind und durch Polyole in ihrer Größe und Form stabilisiert werden.

Ebenso erfindungsgemäß ist die Verwendung von Polyolen, wobei als Polyole bevorzugt Propylenglykol und/oder Glycerin ausgewählt werden, mit einem Gehalt von 15-50 Gew.-%, insbesondere 25 - 40 Gew.-% zur Stabilisierung der Größe und Form von Zuckerkristallen in Reinigungszubereitungen. Bevorzugt ist, dass die Reinigungszubereitungen Tenside, Wasser und wasserlösliche Bestandteile in gelöster und kristalliner Form umfassen, wobei als wasserlöslicher Bestandteil Zucker, von dem ein Teil in Form von Zuckerkristallen vorliegt, enthalten ist und wobei die Kristalle durch den Einsatz von polymeren Strukturanten homogen und stabil in der Zubereitung verteilt sind und durch Polyole in ihrer Größe und Form stabilisiert werden. Ganz besonders bevorzugt ist es, dass die Reinigungszubereitungen als wasserlösliche Bestandteile Saccharose enthalten.

Ebenfalls erfindungsgemäß ist die Verwendung von Polyolen, wobei als Polyole bevorzugt Propylenglykol und/oder Glycerin ausgewählt werden, mit einem Gehalt von 15 bis 50 Gew.-%, insbesondere 25 bis 40 Gew.-% zur Inhibierung des Kristallwachstums von Zuckerkristallen in Reinigungszubereitungen. Bevorzugt ist, dass die Reinigungszubereitungen Tenside, Wasser und wasserlösliche Bestandteile in gelöster und kristalliner Form umfassen, wobei als wasserlöslicher Bestandteil Zucker, von dem ein Teil in Form von Zucker Salzkristallen vorliegt, enthalten ist und wobei die Kristalle durch den Einsatz von polymeren Strukturanten homogen und stabil in der Zubereitung verteilt sind und durch Polyole in ihrer Größe und Form stabilisiert werden. Ganz besonders bevorzugt ist es, dass die Reinigungszubereitungen als wasserlösliche Bestandteile Saccharose enthalten.

Weiterhin erfindungsgemäß ist ein kosmetisches, nicht-therapeutisches Verfahren zur abrasiven Hautreinigung, wobei in einem ersten Schritt Reinigungszubereitungen, die Tenside, Wasser und wasserlösliche Bestandteile in gelöster und kristalliner Form umfassen, wobei als wasserlöslicher Bestandteil Zucker, von dem ein Teil jeweils in Form von Zuckerkristallen vorliegt, enthalten ist und wobei die Kristalle durch den Einsatz von polymeren Strukturanten homogen und stabil in der Zubereitung verteilt sind und durch Polyole in ihrer Größe und Form stabilisiert werden auf die Haut aufgetragen und verteilt werden und in einem zweiten Schritt abgespült werden. Bevorzugt ist es, dass die Reinigungszubereitungen als wasserlösliche Bestandteile Saccharose enthalten.

Auch erfindungsgemäß ist ein kosmetisches, nicht-therapeutisches Verfahren zur Hauterfrischung, wobei in einem ersten Schritt Reinigungszubereitungen, die Tenside, Wasser und wasserlösliche Bestandteile in gelöster und kristalliner Form umfassen, wobei als wasserlöslicher Bestandteil Zucker, von dem ein Teil in Form von Zuckerkristallen vorliegt, enthalten ist und wobei die Kristalle durch den Einsatz von polymeren Strukturanten homogen und stabil in der Zubereitung verteilt sind und durch Polyole in ihrer Größe und Form stabilisiert werden auf die Haut aufgetragen und verteilt werden und in einem zweiten Schritt abgespült werden. Bevorzugt ist es, dass die Reinigungszubereitungen als wasserlösliche Bestandteile Saccharose enthalten.

In der vorliegenden Offenbarung werden die Begriffe Strukturant, polymerer Strukturant, Struktur-Bildner, Gelbildner, Struktur-gebende Substanz oder polymerer Verdicker synonym verwendet.

Die verwendeten polymeren Strukturanten erlauben es, kosmetische Zubereitungen zur Verfügung zu stellen, die Partikel enthalten, die homogen suspendiert sind und es auch bleiben. Die polymeren Strukturanten werden insbesondere zur Hemmung der Sedimentationsneigung von Kohlenhydratkristallen in Kohlenhydrat-gesättigten, tensidhaltigen kosmetischen Reinigungszubereitungen verwendet.

Die erfindungsgemäßen Zubereitungen zeichnen sich durch eine einfache und sichere Herstellung aus. Die Peelingpartikel sind homogen in der Zubereitung verteilt und bleiben es auch. Weiterhin lassen sich die Zubereitungen problemlos aus den entsprechenden Verpackungen entnehmen. Sie weisen ein entsprechend gutes Fließverhalten auf.

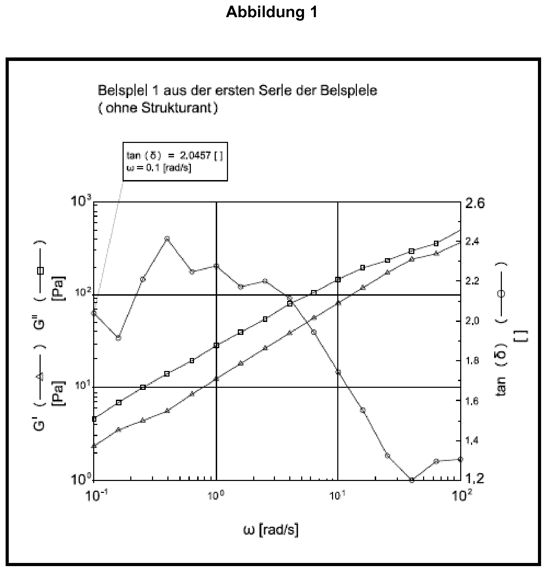

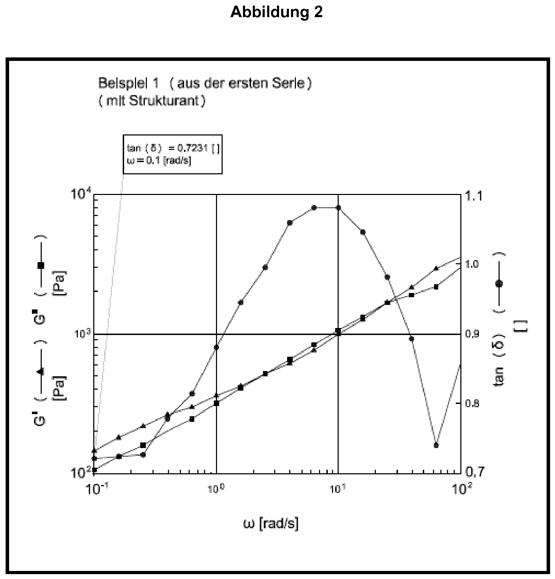

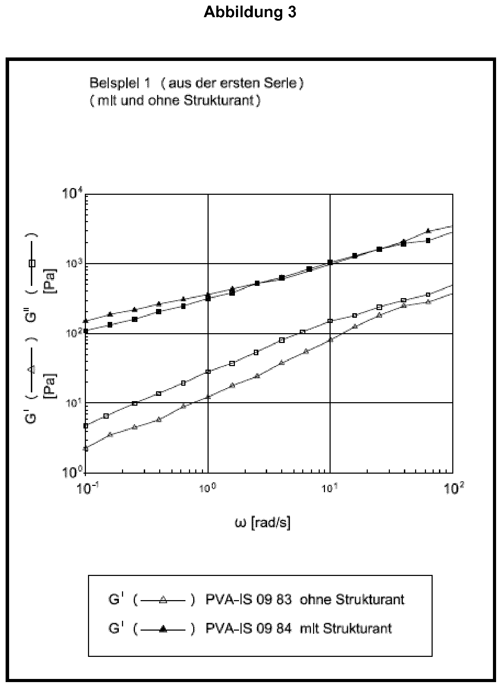

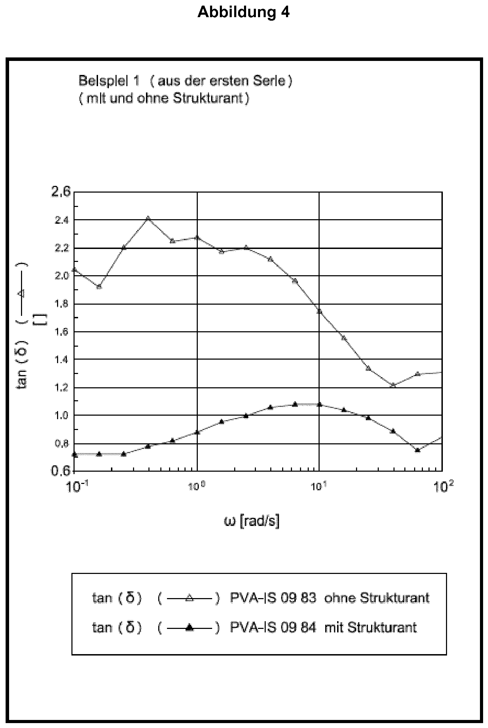

Die Strukturanten haben einen Effekt auf die stabile Suspendierung von Partikel. Dies wird belegt durch die Versuche, die in den Abbildungen 1 bis 4 offenbart sind.

Die Viskositätswerte, die in dieser Offenbarung angeführt werden, sind mit dem Rheomat R123 der Gesellschaft ProRheo bei 25°C gemessen worden. Bei der Messung mit dem Rheomat R123 wird der Rotor des Gerätes blasenfrei bis zur Markierung in die Probe eingetaucht. Für die Messungen wurde der Messkörper 2 verwendet. Weitergehende Informationen zum Rheomat R 123 sind im Internet veröffentlicht, siehe
http://www.prorheo.de/fileadmin/user upload/pdfs/R123.pdf und
http://www.prorheo.de/fileadmin/user upload/pdfs/Bedienung R123 d.pdf.

Um Partikel in der Schwebe zu halten ist neben einer angemessenen Viskosität auch die Ausbildung einer Fließgrenze notwendig.

Als Fließgrenze wird die kritische Schubspannung der Fließkurve angesehen. Sie kann erfindungsgemäß wie folgt ermittelt werden:

Gemessen wird die Fließkurve auf einem schubspannungsgesteuerten Rheometer bei 25°C ± 1 °C mit 20 mm Platte/Platte Geometrie bei einem Spalt zwischen 0,8 mm und 1,2 mm, wobei strukturschonend befüllt wird. Es wird eine geeignete konstante Schubspannungszeitrampe vorgegeben und vor dem Test eine entsprechende Strukturerholungszeit eingehalten und die kritische Schubspannung im Maximum der Fließkurve angegeben.

Eine Fließgrenze bildet sich aus, wenn die Zubereitung neben viskosen Eigenschaften auch einen angemessenen Anteil viskoelastischer Eigenschaften hat. Den Anteil der viskosen und viskoelastischen Eigenschaften kann man mit Frequenzmessungstests bestimmen. Derartige Messungen wurden beispielhaft durchgeführt. Die Abbildungen 1 bis 4 zeigen die Ergebnisse von Frequenzmessungstests zur Ermittlung des Verhältnisses von elastischen zu viskosen Eigenschaften von einer beispielhaften erfindungsgemäßen Zubereitung. Vermessen wurde Beispiel 1 aus der ersten Serie. Die Messungen wurden mit dem Gerät ARES 5 durchgeführt, bei einer Peltiertemperierung von 25°C. Das Messsystem hatte einen Platte-Platte Durchmesser von 50 mm und einen Spalt von 1 mm. Ein Programm von 0,1 -> 100 rad/s wurde verwendet. Bestimmt wurden die elastischen Anteile, beschrieben durch das Speichermodul G', und die viskosen Anteile, beschrieben durch das Verlustmodul G", in der zu vermessenden Probe. Das Verhältnis von G" zu G' ergibt den jeweiligen tan δ Wert. Tan δ Werte unter 1 zeigen an, dass in den vermessenen Zubereitungen die elastischen Eigenschaften überwiegen, ein tan δ Wert von 1 entspricht dem Gelpunkt und tan δ Werte oberhalb von 1 sind ein Zeichen dafür, dass die viskosen Eigenschaften überwiegen.

In den Abbildungen 1 bis 4 sind die Ergebnisse von Frequenzmessungen zur Bestimmung des Verhältnisses von viskosem zu elastischem Anteil in einer beispielhaften Zubereitung mit und ohne polymerem Strukturant (=Gelbildner) dargestellt.

Die erste Abbildung zeigt die Messergebnisse einer Probe ohne polymeren Strukturant (=Gelbildner). Die G"-Werte sind größer als die G'-Werte, d.h. der viskose Anteil in dieser Zubereitung überwiegt. Die tan δ Werte liegen über 1.

Die zweite Abbildung zeigt die Ergebnisse für eine erfindungsgemäße Probe, die einen polymeren Strukturanten (=Gelbildner) enthält. Die erste Probe und die zweite Probe unterscheiden sich durch die Ab- bzw. Anwesenheit des Strukturanten. Die Messergebnisse für die zweite Probe zeigen, dass der elastische Anteil in zwei Frequenzbereichen größer ist als der viskose, dies führt dazu, dass die tan δ Werte fast immer unter 1 liegen.

Die dritte Abbildung vereinigt die Ergebnisse der ersten zwei Abbildungen hinsichtlich der Werte zu G'- und G". Bei der Betrachtung der G'-Kurven wird deutlich, dass in der Probe ohne polymeren Strukturanten (=Gelbildner) deutlich geringere Werte gemessen werden als in der Probe mit polymerem Strukturanten. Das bedeutet, dass der Zusatz des polymeren Strukuranten zur Erhöhung der elastischen Eigenschaften führt.

Die vierte Abbildung vereinigt die tan δ Werte aus den ersten zwei Abbildungen in einer Abbildung. Die tan δ Werte für die Probe mit polymerem Strukturant (=Gelbildner) liegen weitgehend unter 1. Dies bedeutet, dass die elastischen Anteile überwiegen und das wiederum belegt eine Zunahme an Struktur in der Probe. Dies bewirkt, dass in den erfindungsgemäßen Zubereitungen Zuckerkristalle in Schwebe gehalten werden.

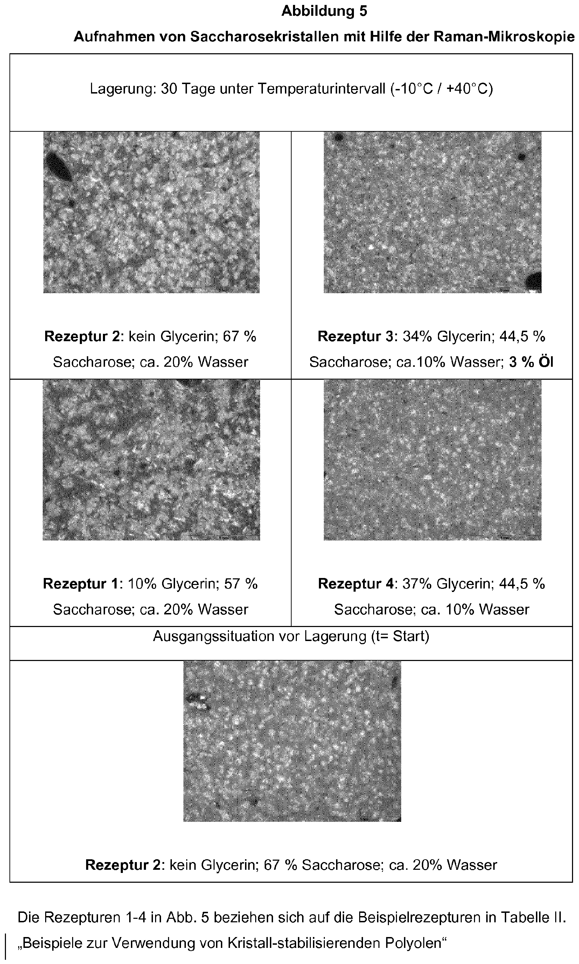

Die Abbildung 5 zeigt die Auswirkungen einer Glycerinzugabe zu einer Reinigungszubereitung in Bezug auf die Hemmung des Kristallwachstum und der Stabilisierung der Größe und Form von Saccharosekristallen. Die Beispiele 1 bis 4 entsprechen den Beispielrezepturen 1 bis 4 der Beispielserie II. "Beispiele zur Verwendung von Kristall-stabilisierenden Polyolen".

Beispiel 2 enthält u.a. 10 Gew.-% Glycerin, 57 Gew.-% Saccharose und ca. 20 Gew.-% Wasser. In dem Abbildungsteil Ausgangssituation vor der Lagerung (t=Start) wird eine Aufnahme einer Reinigungszubereitung mit einem Raman-Mikroskop gezeigt. Die Kristalle erscheinen klein und regelmäßig. Die Sensorik einer derartigen Reinigungszubereitung ist angenehm und die Kristalle lösen sich beim Abwaschschritt gut und zügig auf und hinterlassen praktisch keine Rückstände.

Der zweite Teil der Abbildung 5, Lagerung 30 Tage unter Temperaturintervall, zeigt Aufnahmen von Reinigungszubereitungen mit dem Raman-Mikroskop nach 30 Tagen Lagerung bei wechselnden Temperaturbedingungen, wobei wechselnde Lagerungsbedingungen bedeutet, dass die Temperatur im Tagesverlauf von -10°C auf +40°C und wieder zurück auf -10°C wechselt. Mit diesen wechselnden Lagerbedingungen sollte der Einfluss von Temperaturschwankungen auf die Kristallstruktur und -größe untersucht werden, um das maximale Kristallwachstum während der Lebensdauer eines kosmetischen Produktes von etwa 3 Jahren im Bereich normaler Klimaschwankungen zu prognostizieren. Die Aufnahme zum gelagerten Beispiel 2 zeigt im Vergleich zur Aufnahme der Ausgangssituation deutlich größere Kristalle, die auch unregelmäßiger erscheinen. Die Aufnahme zu Beispiel 1, wobei Beispiel 1 u.a. 10 Gew.-% Glycerin, 57 Gew.-% Saccharose und etwa 20 Gew.-% Wasser enthält, zeigt eine Verbesserung gegenüber Beispiel 2, die Kristalle erscheinen jedoch noch größer als in der Ausgangslösung und unregelmäßiger. Die Aufnahme zu Beispiel 3, wobei Beispiel 3 u.a. 33,7 Gew.-% Glycerin, 44,5 Gew.-% Saccharose, etwa 10 Gew.-% Wasser und 3 Gew.-% Öl enthält, zeigt wiederum eine deutliche Verbesserung gegenüber den Aufnahmen zu Beispiel 1 und 2. Die Kristalle erscheinen kleiner und regelmäßiger geformt. Die Aufnahme zu Beispiel 4, wobei Beispiel 4 u.a. 36,7 Gew.-% Glycerin, 44,5 Gew.-% Saccharose, ca. 10 Gew.-% Wasser und kein Öl enthält, zeigt Kristalle, die der Ausgangsrezeptur, Ausgangssituation Beispiel 2, sehr ähnlich sind in Bezug auf Größe und Form und nur einen geringen Größenzuwachs aufweisen.

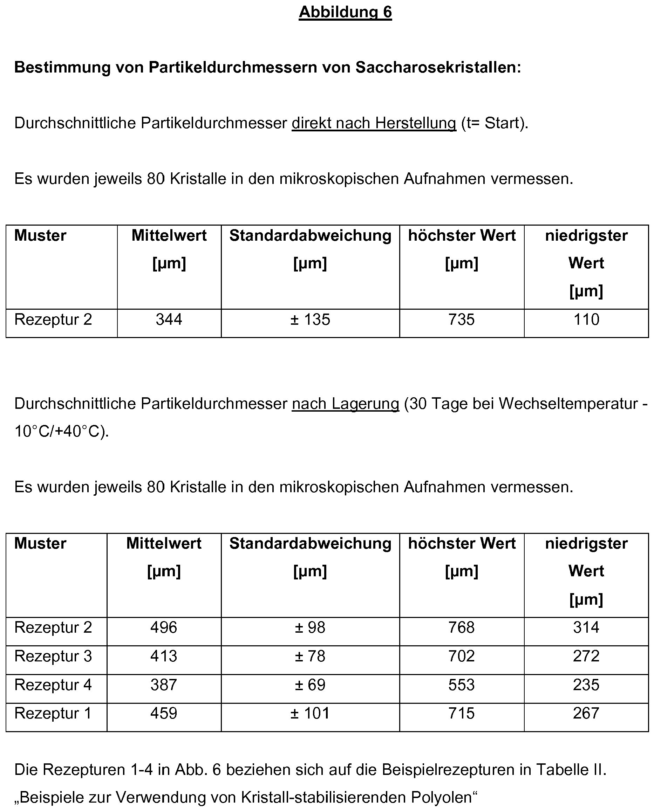

Abbildung 6 listet eine Bestimmung des Partikeldurchmessers auf. Zur Bestimmung der Partikeldurchmesser wurden jeweils 80 Kristalle in den Raman-Mikroskopie-Aufnahmen der jeweiligen Rezepturen vermessen. Die Ergebnisse, die durch die Fotos von der Raman-Mikroskopie sichtbar wurden, werden durch die Angaben zu den Partikeldurchmessern bestätigt. Das Beispiel 2, das kein Glycerin enthält, weist nach der Lagerung die größten Kristalle auf. Die Kristalle in Reinigungszubereitungen, die Glycerin enthalten, sind nach 30 Tagen Lagerung deutlich kleiner, bei 10 Gew.-% Glycerin (Beispiel 1) durchschnittlich 459 µm, bei 34 Gew.-% Glycerin (Beispiel 3) durchschnittlich 413 µm und bei 37 Gew.-% Glycerin (Beispiel 4) bei durchschnittlich 387 µm.

Diese Untersuchungen belegen, dass Glycerin das Kristallwachstum von Saccharosekristallen hemmt und zu einer Stabilisierung der Saccharosekristalle in Bezug auf Größe und Regelmäßigkeit in der Form beiträgt. Bei den Beispielen 3 und 4 wurde auf eine Wasserzugabe verzichtet, der Wasseranteil in der Rezeptur stammt aus Bestandteilen der Rezeptur, die in Wasser gelöst zugesetzt werden, z. B Cocosulfat und Cocoglucosid.

Darüber hinaus ist es möglich durch den Einsatz von Glycerin die Menge an Saccharose zu reduzieren. Dies wird deutlich in Vergleich der Beispiele 1, 2 und 3. Ohne Zusatz von Glycerin (Beispiel 2) wurden 67 Gew.-% Saccharose zugefügt um eine ausreichende Menge an Saccharosekristallen in der Reinigungszubereitung zu erhalten. Bei Zusatz von 10 Gew.-% Glycerin (Beispiel 1) konnte die Saccharosemenge auf 57 Gew.-% verringert werden und bei einer Zugabe von 34 Gew.-% Glycerin waren 44,5 Gew.-% Saccharose ausreichend für eine hinreichenden Menge von Peelingkristallen in der Zubereitung.

Die *Tenside,* die in den erfindungsgemäßen Zubereitungen Verwendung finden, können anionische und/oder amphotere und/oder kationische und/oder nichtionische Tenside sein.

Vorteilhaft zu verwendende anionische Tenside sind
Acylaminosäuren (und deren Salze), wie
1. Acylglutamate, beispielsweise Natriumacylglutamat, Natrium Cocoylglutamat, Di-TEApalmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat.

Carbonsäuren und Derivate, wie
1. Carbonsäuren, beispielsweise Laurinsäure, Aluminiumstearat, Magnesiumalkanolat und Zinkundecylenat, Stearinsäure/-salz, Palmitinsäure/-salz,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-Phosphat,
Sulfonsäuren und Salze, wie
1. Acyl-isethionate, z.B. Natrium-/ Ammoniumcocoyl-isethionat, Natrium Lauryl Methylisethionate,
2. Alkylarylsulfonate,
3. Alkylsulfonate, beispielsweise Natriumcocosmonoglyceridsulfat, Natrium C12-14 Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
1. Alkylethersulfat mit unterschiedlichen Ethoxylierungsgraden und deren Gemische, beispielsweise Natrium-, Ammonium-, Magnesium-, MIPA-, TIPA-Laureth-X-sulfat, Natriummyreth-X-sulfat und Natrium C12-13-Pareth-X-sulfat, mit X = 1-5 Ethoxygruppen.
2. Alkylsulfate, beispielsweise Natrium-, Ammonium- und TEA-Laurylsulfat, Natrium-Ammonium- und TEA-Cocosulfat.

Vorteilhaft zu verwendende kationische Tenside sind
1. Alkylamine,
2. Alkylimidazole,ethoxylierte Amine
3. Quaternäre Tenside, beispielsweise Cetyl Trimethylammoniumhalogenid.
4. Esterquats, beispielsweise Dicocoylethyl Hydroxyethylmoium Methosulfate und
5. Amidquats, beispielsweise Palmitamidopropyltrimonium Chlorid.

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkylamidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Dinatrium Cocoamphodiacetat, Dinatrium Cocoamphomonoacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.
3. Betaine, beispielsweise Coco Betaine, Cocoamidopropyl Betaine,
4. Sultaine, beispielsweise Lauryl Hydroxy Sultaine.

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, Laureth-X mit X = 2 bis 10, wobei X Ethoxygruppe bedeutet, ethoxylierte/ propoxylierte Ester, ethoxylierte/ propoxylierte Glycerinester, PEG-200 Hydrogenated Glyceryl Palmat, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

*Suspendierte Partikel* sind partikuläre Strukturen, die in die Reinigungszubereitungen eingearbeitet werden können. Diese Partikel können verschiedenartige Funktionen erfüllen.

Wenn diese suspendierten Partikel eine Peelingfunktion in der Zubereitung erfüllen, werden sie als Peelingpartikel oder Peelingkörper bezeichnet. Die Begriffe Peelingpartikel oder Peelingkörper werden in dieser Offenbarung synonym verwendet. Es handelt sich um feste und/oder kristalline Strukturen, die eine Peelingwirkung hervorrufen. Unter den erfindungsgemäßen Peelingpartikeln werden die ungelösten Bestandteile von Kohlenhydraten, beispielsweise Saccharose, verstanden, die zumindest teilweise in fester und/oder kristalliner Form vorliegen. Dies wird erreicht durch eine so hohe Kohlenhydratkonzentration, dass sich nicht alle Kohlenhydratmoleküle in der erfindungsgemäßen Zubereitung lösen können, sondern in fester und/oder kristalliner Form vorliegen. Dies bedeutet, dass beispielsweise im Falle eines Einsatzes von Saccharose die entsprechenden Kristalle in der Reinigungszubereitung vorliegen. Die Peelingpartikel in Form dieser Kristalle kommen bei Anwendung während des Dusch- oder Waschvorgangs mit Wasser in Kontakt, beispielsweise beim Abduschen oder Abwaschen des Schaums und der Reinigungszubereitung am Ende des Reinigungsvorgangs. Hierdurch ist ein Auflösen der Kristalle gewährleistet, was zu einem abnehmenden Peelingeffekt führt. Es bleiben keine oder praktisch keine Rückstände der Peelingsubstanzen in Waschbecken oder Behandlungsvorrichtungen zurück. Praktisch keine Rückstände bedeutet, dass in einzelnen wenigen Fällen äußerst geringe Spuren der Reinigungszubereitung nach dem Waschvorgang auf Waschtischen oder Anwendungseinrichtungen sichtbar sind, die jedoch auf einfache Weise durch Abspülen oder Wegwischen entfernbar sind.

Zusätzliche Peelingmittel sind aufgrund der erfindungsgemäßen Ausführungen überflüssig. Selbstverständlich ist aber eine Zugabe zusätzlicher Peelingmittel möglich. Die Peelingmittel werden dann vorteilhaft gewählt aus der Gruppe natürlicher oder naturbasierter Peelingmittel wie beispielsweise Tonerde, Sand, zerstoßene oder zermahlene Kerne von Walnusschalen, Aprikosen-, Pfirsich- und/oder Mandelkernen, Peelingpartikel aus wachsartigen, festen Grundlagen wie gehärtetes Rizinusöl, Jojobaöl, Carnaubawachs, Candelellawachs, gehärtetes Sonnenblumenöl, Reiskeimöl, gehärtetes Sojaöl und Paraffin. Ebenso sind Peelingpartikel aus Kieselsäure und Silikaten oder Polylactiden denkbar. Auch möglich, wenngleich nicht erwünscht, ist der Einsatz vollsynthetischer Peelingmittel.

Um den erfindungsgemäßen Zubereitungen rückfettende Eigenschaften zu verleihen können beispielsweise *Öle* in die erfindungsgemäßen Zubereitungen eingearbeitet werden.

Die Öle können ausgewählt werden aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* erhältlich ist.

Es ist ferner bevorzugt, die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C12-13-Alkyllactat, Di-C12-13-Alkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen Formulierungen einen Gehalt an C12-15-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Bevorzugt sind unpolare Öle, wie beispielsweise verzweigte oder unverzweigte Kohlenwasserstoffe und -wachse, insbesondere Mineralöl, Vaseline (Petrolatum), Paraffinöl, Squalan und Squalen, Polyolefine, hydrogenierte Polyisobutene und Isohexadekan.

Besonders vorteilhaft ist beispielsweise die verwendung von Coco-Caprylate/Caprate.

Erfindungsgemäße Zubereitungen können ein oder mehrere *polymere Strukturanten* enthalten. Die polymeren Strukturanten können vorteilhaft gewählt werden aus der Gruppe der Gummen, Polysaccharide, Cellulosederivate, Schichtsilikate, Polyacrylate und/oder anderen Polymeren.

Zu den Gummen zählt man Pflanzen- oder Baumsäfte, die an der Luft erhärten und Harze bilden oder Extrakte aus Wasserpflanzen. Aus dieser Gruppe können beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi ausgewählt werden; besonders vorteilhaft ist Xanthangummi.

Weiterhin vorteilhaft ist die Verwendung von derivatisierten Gummen wie z. B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z. B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z. B. Stearylalkonium Hektorite.

Weiterhin können auch Kieselsäuregele verwendet werden.

Vorteilhaft sind außerdem Taurate, z. B. Ammonium Acryloyldimethyltaurat / VP Copolymer.

Unter den Polyacrylaten befinden sich z.B. Carbopol Typen der Firma Lubrizol (Carbopol 980, 981, 1382, 5984, 2984, ETD 2001, ETD 2020, ETD 2050, Ultrez-10 oder Pemulen TR1 & TR2), Acrylates Copolymer (Handelsname Carbopol Aqua SF-1) oder Acrylates Crosspolymer-4 (Handelsname: Carbopol Aqua SF-2).

Unter den Polymeren befinden sich z. B. Polyacrylamide (Seppigel 305), Polyvinylalkohole, PVP, PVP / VA Copolymere, Polyglycole.

Erfindungsgemäß besonders bevorzugt ist ein Acrylic Acid/VP Crosspolymer von der Firma ASI für Reinigungszubereitungen enthaltend Kristall-stabilisierende Polyole.

Erfindungsgemäße Zubereitungen können einen oder mehrere *Emulgatoren* enthalten. Bevorzugte Emulgatoren sind W/O-Emulgatoren.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter oder unverzweigter Alkancarbonsäuren einer Kettenlänge von 8 bis 24, insbesondere 12 bis 20 Kohlenstoffatomen, Diglycerinester gesättigter und/oder ungesättigter, verzweigter oder unverzweigter Alkancarbonsäuren einer Kettenlänge von 8 bis 24, insbesondere 12 bis 20 Kohlenstoffatomen, Monoglycerinether gesättigter und/oder ungesättigter, verzweigter oder unverzweigter Alkohole einer Kettenlänge von 8 bis 24, insbesondere 12 bis 20 Kohlenstoffatomen, Diglycerinether gesättigter und/oder ungesättigter, verzweigter oder unverzweigter Alkohole einer Kettenlänge von 8 bis 24, insbesondere 12 bis 20 Kohlenstoffatomen, Polypropylenglykolester gesättigter und/oder ungesättigter, verzweigter oder unverzweigter Alkancarbonsäuren einer Kettenlänge von 8 bis 24, insbesondere 12 bis 20 Kohlenstoffatomen, Polyglycerylester gesättigter und/oder ungesättigter, verzweigter oder unverzweigter Alkancarbonsäuren einer Kettenlänge von 8 bis 24, insbesondere 12 bis 20 Kohlenstoffatomen, Sorbitanester gesättigter und/oder ungesättigter, verzweigter oder unverzweigter Alkancarbonsäuren einer Kettenlänge von 8 bis 24, insbesondere 12 bis 20 Kohlenstoffatomen.

Erfindungsgemäß bevorzugt ist beispielsweise Glyceryl-Oleat.

Bevorzugte W/O-Emulgatoren sind verzweigte oder unverzweigte, gesättigte oder ungesättigte Fettsäuren mit 12 bis 26 Kohlenstoffatomen, Polyglyceryl-3 Diisostearat, Polyglyceryl-4 Isostearat, Polyglyceryl-2 Dipolyhydroxystearat, Cetyl PEG/PPG-10-1-Dimethicone, PEG-30 Dipolyhydroxystearat, PEG-40 Sorbitanperisostearat, Cetyldimethiconecopolyol, PEG-7 Hydrogenated Castor Oil, PEG 45/Dodecylglycolcopolymer, PEG 22/Dodeceylglycolcopolymer, Pentaerythritylisostearat, Isostearyldiglycerylsuccinat, Sorbitanisostearat, Polyglyceryl-2 Sesquiisostearat, Glycerylisostearat, Sorbitanstearat, Glycerylstearat, PEG-25 Hydrogenated Castor Oil, PEG-40 Sorbitanperoleat, Sorbitanoleat, PEG-40 Sorbitanperisostearat, Polyglyceryl-3 Oleat, Polyglyceryl-2 Sesquioleat und Polyglyceryl-4 Isostearat.

Besonders bevorzugte W/O-Emulgatoren sind ethoxylierte Fettalkohole, insbesondere Laureth-2.

Erfindungsgemäß sind auch Substanzen, die üblicherweise eingesetzt werden, um den pH-Wert der erfindungsgemäßen Zubereitungen einzustellen und stabil zu halten. Vorteilhaft können diese Substanzen ausgewählt werden aus der Gruppe Zitronensäure und Milchsäure und/oder aus der Gruppe Aminomethylpropanol, Natronlauge, Kalilauge und Triethanolamin.

Optional können, wenn erforderlich, die in der Kosmetik üblichen Zusatz- und Hilfsstoffe in die Zubereitungen eingearbeitet werden, wie z. B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Geschmacksstoffe, Vergällungsmittel, Parfums, weichmachende Substanzen, anfeuchtende und/oder feuchthaltende Substanzen, Antioxidantien, UV-Filtersubstanzen, Sensorikadditive, Filmbildner, Wirkstoffe oder andere übliche Bestandteile einer kosmetischen Formulierung wie Alkohole, Polyole, Schaumstabilisatoren, Elektrolyte, organische Lösungsmittel oder Silikonderivate.

### Beispiele:

### I. Beispiele zur Verwendung von Polymeren Strukturanten:

**Erste Serie an Beispielen (wird auf der nächsten Seite fortgesetzt):**

| **Ansatznummer** | **1** | **2** | **3** | **4** | **5** | **6** | **7** | **8** |
|---|---|---|---|---|---|---|---|---|
| **INCI** | **m [%]** | **m [%]** | **m [%]** | **m [%]** | **m [%]** | **m [%]** | **m [%]** | **m [%]** |
| Aqua | 13,64 | 16,14 | 15,64 | 15,14 | 23,21 | 25,22 | 25,32 | 27,82 |
| Paraffinum Liquidum | 5,00 | 5,00 | 5,00 | 5,00 | | | | |
| Aqua + Sodium Hydroxide (45%) | | | | | | | | |
| Sodium Chloride | 1,50 | 2,00 | 2,50 | 3,00 | | | | |
| Sodium Myreth Sulfate (71%) | 7,70 | 7,70 | 7,70 | 7,70 | | | | |
| Sodium Laureth Sulfate (70%) | | | | | | | | |
| Laureth-2 (99,85%) | 1,20 | 1,20 | 1,20 | 1,20 | | | | |
| Lauryl Glucoside (51,5%) | | | | | | | | |
| Coco-Caprylate/Caprate | | | | | 2,50 | 2,50 | 2,50 | |
| Cocam idopropyl Betaine + Glycerin (34%) | | | | | | | | |
| Decyl Glucoside (53%) | 2,81 | 2,81 | 2,81 | 2,81 | | | | |
| Aminomethyl Propanol (95%) | 0,00 | | | | | | | |
| Coco-Glucoside (52%) | | | | | 5,33 | 2,26 | 2,26 | 2,26 |
| Parfum | 0,15 | 0,15 | 0,15 | 0,15 | | | | |
| Coco-Glucoside + Glyceryl Oleate (70%) | | | | | 0,68 | 0,91 | 0,91 | 0,91 |
| Disodium Cocoyl Glutamate (25%) | | | | | | | | |
| **Acrylic Acid/VP Crosspolymer** | **1,00** | **1,00** | **1,00** | **1,00** | **1,20** | **1,00** | **0,90** | **0,90** |
| Ammonium Lauryl Sulfate (30%) | | | | | | | | |
| Sucrose | 67,00 | 64,00 | 64,00 | 64,00 | 64,00 | 64,00 | 64,00 | 64,00 |
| Parfum | | | | | | | | |
| Sodium Coco-Sulfate (92,5%) | | | | | 3,08 | 4,11 | 4,11 | 4,11 |
| Summen: | 100,00 | 100,00 | 100,00 | 100,00 | 100,00 | 100,00 | 100,00 | 100,00 |

Die Werte in Klammern hinter den Substanznamen beziehen sich auf die Aktivgehalte.

**Erste Serie an Beispielen (Fortsetzung der vorangehenden Seite):**

| **Ansatznummer** | **9** | **10** | **11** | **12** | **13** | **14** | **15** | **16** | **17** |
|---|---|---|---|---|---|---|---|---|---|
| **INCI** | **m [%]** | **m [%]** | **m [%]** | **m [%]** | **m [%]** | **m [%]** | **m [%]** | **m [%]** | **m [%]** |
| Aqua | 9,36 | 16,07 | 15,67 | 22,67 | 16,74 | 16,84 | 16,35 | 16,45 | 22,97 |
| Paraffinum Liquidum | | 5,00 | 5,00 | | 5,00 | 5,00 | 5,00 | 5,00 | |
| Aqua + Sodium Hydroxide (45%) | 0,58 | | | | | | | | |
| Sodium Chloride | | 1,50 | | | 1,50 | 1,50 | | | |
| Sodium Myreth Sulfate (71%) | | 7,70 | | | 7,70 | 7,70 | | | |
| Sodium Laureth Sulfate (70%) | | | 5,71 | | | | 5,71 | 5,71 | |
| Laureth-2 (99,85%) | | 1,20 | | | 1,20 | 1,20 | | | |
| Lauryl Glucoside (51,5%) | 5,48 | | | | | | | | |
| Coco-Caprylate/Caprat e | 3,00 | | | 5,00 | | | | | 5,00 |
| Cocam idopropyl Betaine + Glycerin (34%) | 9,15 | | | | | | | | |
| Decyl Glucoside (53%) | | 2,81 | | | 2,81 | 2,81 | | | |
| Aminomethyl Propanol (95%) | | 0,57 | 0,58 | | | | | | 0,34 |
| Coco-Glucoside (52%) | | | 7,69 | 2,26 | | | 7,69 | 7,69 | 2,02 |
| Parfum | | 0,15 | 0,15 | 0,15 | 0,15 | 0,15 | 0,15 | 0,15 | |
| Coco-Glucoside + Glyceryl Oleate (70%) | | | | 0,91 | | | | | 0,79 |
| Disodium Cocoyl Glutamate (25%) | 2,76 | | | | | | | | |
| **Acrylic Acid/VP Crosspolymer** | **1,10** | **1,00** | **1,20** | **0,90** | **0,90** | **0,80** | **1,10** | **1,00** | **0,90** |
| Ammonium Lauryl Sulfate (30%) | 4,57 | | | | | | | | |
| Sucrose | 64,00 | 64,00 | 64,00 | 64,00 | 64,00 | 64,00 | 64,00 | 64,00 | 64,00 |
| Parfum | | | | | | | | | 0,30 |
| Sodium Coco-Sulfate (92,5%) | | | | 4,11 | | | | | 3,68 |
| Summen: | 100,0 0 | 100,0 0 | 100,0 0 | 100,0 0 | 100,0 0 | 100,0 0 | 100,0 0 | 100,0 0 | 100,0 0 |

Die Werte in Klammern hinter den Substanznamen beziehen sich auf die Aktivgehalte.

**Zweite Serie an Beispielen:**

| **Ansatznummer** | **1** | **2** | **3** | **4** | **5** | **6** |
|---|---|---|---|---|---|---|
| **INCI** | **m [%]** | **m [%]** | **m [%]** | **m [%]** | **m [%]** | **m [%]** |
| Aqua | 14,27 | 14,13 | 6,41 | 16,99 | 9,41 | 12,49 |
| Sodium Laureth Sulfate (70%) | 5,4 | 5,61 | - | 6,86 | - | 5,71 |
| Cocamidopropyl Betaine + Glycerin (34%) | 9,41 | 8 | 3,79 | - | - | - |
| PEG-7 Glyceryl Cocoate | 1,02 | 1,06 | - | - | - | - |
| Disodium Cocoyl Glutamate (25%) | - | 1,2 | - | - | - | - |
| Coco Glucoside (52%) | - | - | 4,71 | 6,15 | 7,69 | - |
| Sodium Cocoamphoacetate | - | - | 2,92 | - | - | - |
| Ammonium Lauryl Sulfate (30%) | - | - | 11,07 | - | - | - |
| Coco Betaine | - | - | - | - | 12,9 | 12,9 |
| Sodium Chloride | 1,5 | 1,5 | 1,5 | 1,5 | 1,5 | 1,5 |
| Coco-Caprylate/Caprate | 3 | 3 | 4 | 3 | 3 | 2 |
| Sucrose | 64 | 64 | 64 | 64 | 64 | 64 |
| Citric Acid | qs | qs | qs | qs | qs | qs |
| Aminomethyl Propanol (95%) | qs | qs | qs | qs | qs | qs |
| **Acrylic Acid/Vp Crosspolymer** | **0,9** | **1** | **1,1** | **1** | **1** | **0,9** |
| Gesamt | 100 | 100 | 100 | 100 | 100 | 100 |

Die Werte in Klammern hinter den Substanznamen beziehen sich auf die Aktivgehalte.

**Dritte Serie an Beispielen (wird auf der folgenden Seite fortgesetzt):**

| **Ansatznummer** | **1** | **2** | **3** | **4** | **5** | **6** | **7** | **8** |
|---|---|---|---|---|---|---|---|---|
| **INCI** | **m [%]** | **m [%]** | **m [%]** | **m [%]** | **m [%]** | **m [%]** | **m [%]** | **m [%]** |
| Aqua | 13,14 | 11,60 | 15,24 | 18,34 | 18,24 | 18,14 | 17,94 | 18,54 |
| Citric Acid | | 0,00 | | | | | | |
| Paraffinum Liquidum | 5,00 | 5,00 | 5,00 | 5,00 | 5,00 | 5,00 | 5,00 | 5,00 |
| Aqua + Sodium Hydroxide (45%) | 0,02 | 0,18 | | | | | | |
| Xanthan Gum | | | | | | | | |
| Sodium Chloride | 1,50 | 1,50 | | | | | | |
| **Acrylates/C10-30 Alkyl Acrylate Crosspolymer** | | | **0,90** | **0,80** | **0,90** | **1,00** | | **0,60** |
| **Carbomer** | | | | | | | **1,20** | |
| Sodium Myreth Sulfate (71 %) | 7,70 | 8,10 | 7,70 | 7,70 | 7,70 | 7,70 | 7,70 | 7,70 |
| Sodium Laureth Sulfate (70%) | | | | | | | | |
| Laureth-2 (99,85%) | 1,20 | 1,26 | 1,20 | 1,20 | 1,20 | 1,20 | 1,20 | 1,20 |
| Decyl Glucoside (53%) | 2,81 | 2,96 | 2,81 | 2,81 | 2,81 | 2,81 | 2,81 | 2,81 |
| Coco-Glucoside (52%) | | | | | | | | |
| Parfum | 0,15 | 0,15 | 0,15 | 0,15 | 0,15 | 0,15 | 0,15 | 0,15 |
| **Ammonium Acryloyldimethyltaurate/VP Copolymer** | **1,50** | **1,75** | | | | | | |
| Sucrose | 66,98 | 67,50 | 67,00 | 64,00 | 64,00 | 64,00 | 64,00 | 64,00 |
| Summen: | 100,00 | 100,00 | 100,00 | 100,00 | 100,00 | 100,00 | 100,00 | 100,00 |

Die Werte in Klammern hinter den Substanznamen beziehen sich auf die Aktivgehalte.

**Dritte Serie an Beispielen (Fortsetzung der vorangehenden Seite):**

| **Ansatznummer** | **9** | **10** | **11** | **12** | **13** | **14** | **15** | **16** |
|---|---|---|---|---|---|---|---|---|
| **INCI** | **m [%]** | **m [%]** | **m [%]** | **m [%]** | **m [%]** | **m [%]** | **m [%]** | **m [%]** |
| Aqua | 18,44 | 18,34 | 18,54 | 18,44 | 18,34 | 18,24 | 18,14 | 16,45 |
| Citric Acid | | | | | | | | |
| Paraffinum Liquidum | 5,00 | 5,00 | 5,00 | 5,00 | 5,00 | 5,00 | 5,00 | 5,00 |
| Aqua + Sodium Hydroxide (45%) | | | | | | | | |
| Xanthan Gum | | | 0,60 | 0,70 | | 0,90 | 1,00 | |
| Sodium Chloride | | | | | | | | |
| **Acrylates/C10-30 Alkyl Acrylate Crosspolymer** | 0,70 | **0,80** | | | | | | **1,00** |
| **Carbomer** | | | | | | | | |
| Sodium Myreth Sulfate (71%) | 7,70 | 7,70 | 7,70 | 7,70 | 7,70 | 7,70 | 7,70 | |
| Sodium Laureth Sulfate (70%) | | | | | | | | 5,71 |
| Laureth-2 (99,85%) | 1,20 | 1,20 | 1,20 | 1,20 | 1,20 | 1,20 | 1,20 | |
| Decyl Glucoside (53%) | 2,81 | 2,81 | 2,81 | 2,81 | 2,81 | 2,81 | 2,81 | |
| Coco-Glucoside (52%) | | | | | | | | 7,69 |
| Parfum | 0,15 | 0,15 | 0,15 | 0,15 | 0,15 | 0,15 | 0,15 | 0,15 |
| **Ammonium Acryloyldimethyltaurate/ VP Copolymer** | | | | | | | | |
| Sucrose | 64,00 | 64,00 | 64,00 | 64,00 | 64,00 | 64,00 | 64,00 | 64,00 |
| Summen: | 100,0 0 | 100,0 0 | 100,0 0 | 100,0 0 | 100,0 0 | 100,0 0 | 100,0 0 | 100,0 0 |

Die Werte in Klammern hinter den Substanznamen beziehen sich auf die Aktivgehalte.

**Vierte Serie an Beispielen:**

| **Ansatznummer** | **1*** | **2*** | **3*** |
|---|---|---|---|
| **INCI** | **m [%]** | **m [%]** | **m [%]** |
| Aqua | 21,35 | 12,67 | 13,40 |
| Sodium Chloride | 50,00 | 60,00 | 60,00 |
| Lauryl Glucoside (51,5%) | | 5,83 | |
| Coco-Caprylate/Caprate | 3,00 | 5,00 | 3,00 |
| Cocamidopropyl Betaine + Glycerin (34%) | | 7,35 | |
| Aminomethyl Propanol (95%) | 0,28 | 0,30 | 0,23 |
| Coco-Glucoside (52%) | 2,02 | | 2,02 |
| Coco-Glucoside + Glyceryl Oleate (70%) | 0,79 | | 0,79 |
| Maris Sal | 10,00 | | |
| Disodium Cocoyl Glutamate (25%) | | | 8,00 |
| **Acrylic Acid/VP Crosspolymer** | **0,50** | **0,50** | **0,50** |
| Ammonium Lauryl Sulfate (30%) | | 8,35 | |
| Parfum | 0,50 | | 0,50 |
| Coco-Sulfate (30% in H₂O) | 11,56 | | 11,56 |
| | | | |
| Summen: | 100,00 | 100,00 | 100,00 |

Die Werte in Klammern hinter den Substanznamen beziehen sich auf die Aktivgehalte. * nicht erfindungsgemäßes Beispiel.

### II. Beispiele zur Verwendung von Kristall-stabilisierenden Polyolen

**Zuckerhaltige Waschaele**

| **Beispiele:** | **1** | **2** | **3** | **4** |
|---|---|---|---|---|
| **INCI** | **m [%]** | **m [%]** | **m [%]** | **m [%]** |
| Coco-Sulfate (30% in H₂O) | 11,6 | 11,6 | 11,6 | 11,6 |
| Coco-Glucoside (52% in H₂O) | 2 | 2 | 2 | 2 |
| Coco-Glucoside + Glyceryl Oleate (70%) | 0,8 | 0,8 | 0,8 | 0,8 |
| Acrylic Acid/VP Crosspolymer | 0,5 | 0,5 | 0,5 | 0,5 |
| Aminomethyl Propanol (95%) | 0,3 | 0,3 | 0,3 | 0,3 |
| Coco-Caprylate/Caprate | 3 | 3 | 3 | - |
| **Glycerin (99%)** | **10** | **-** | **33,7** | **36,7** |
| **Sucrose** | **57** | **67** | **44,5** | **44,5** |
| Sodium Chloride | 3 | 3 | 3 | 3 |
| Parfum | 0,6 | 0,6 | 0,6 | 0,6 |
| Aqua | 11,2 | 11,2 | | |
| | | | | |
| Summen: | 100,00 | 100,00 | 100,00 | 100,00 |

Die Angaben in den Klammern beziehen sich auf die Aktivgehalte.

| **Beispiele:** | **5** | **6** |
|---|---|---|
| **INCI** | **m [%]** | **m [%]** |
| Coco-Sulfate (30% in H₂O) | 11,6 | 11,6 |
| Coco-Glucoside (52% in H₂O) | 2 | 2 |
| Coco-Glucoside+ Glyceryl Oleate (70%) | 0,8 | 08 |
| Acrylic Acid/VP Crosspolymer | 1,2 | 0,5 |
| Aminomethyl Propanol (95%) | 0,6 | 0,3 |
| Coco-Caprylate/Caprate | 3 | 3 |
| **Glycerin (99%)** | **27,8** | **26,2** |
| Propylene Glycol | 5 | |
| **Sucrose** | **46,5** | **46,5** |
| Sodium Chloride | 0,9 | 6 |
| Parfum | 0,6 | 0,6 |
| Aqua | | 2,5 |
| | | |
| Summen: | 100,00 | 100,00 |

Die Angaben in den Klammern beziehen sich auf die Aktivgehalte.

## Patentansprüche

1. Reinigungszubereitungen umfassend
Tenside, Wasser und
wasserlösliche Bestandteile in gelöster und kristalliner Form,
**dadurch gekennzeichnet, dass** als wasserlösliche Bestandteile Zucker, wobei ein Teil davon in Form von Zuckerkristallen vorliegt, enthalten ist und
wobei die Kristalle durch den Einsatz von polymeren Strukturanten homogen und stabil in der Zubereitung verteilt sind und durch Polyole mit einem Gehalt ≥ 15 Gew.-%, bezogen auf das Gesamtgewicht der Zubereitung, in ihrer Größe und Form stabilisiert werden, wobei die Polyole ausgewählt werden aus der Gruppe Glycerin und Propylenglycol.

2. Kosmetische Reinigungszubereitungen nach Anspruch 1 **dadurch gekennzeichnet, dass** es sich bei den Zuckern um Mono- und/oder Di- und/oder Oligosaccharide handelt.

3. Kosmetische Reinigungszubereitungen nach wenigstens einem der vorangehenden Ansprüche **dadurch gekennzeichnet, dass** es sich bei den Zuckern um Disaccharide, insbesondere Saccharose, handelt.

4. Kosmetische Reinigungszubereitungen nach wenigstens einem der vorangehenden Ansprüche, **dadurch gekennzeichnet, dass** die Zucker mit einem Gehalt von 10 bis 90 Gew.-%, bevorzugt 15 bis 80 Gew.-%, insbesondere bevorzugt 20 bis 70 Gew.-%, bezogen auf das Gesamtgewicht der Zubereitung vorhanden sind.

5. Reinigungszubereitung nach wenigstens einem der Ansprüche 1 bis 4, **dadurch gekennzeichnet, dass** die polymeren Strukturanten ausgewählt werden aus der Gruppe der Celluloseether, Polyacrylate und Polyacrylat Copolymere.

6. Reinigungszubereitung nach Anspruch 5, **dadurch gekennzeichnet, dass** die polymeren Strukturanten Polyacrylat Copolymere sind, wobei Acrylic Acid/VP Crosspolymer, Acrylates Copolymer und Acrylates Crosspolymer-4 bevorzugt sind.

7. Reinigungszubereitung nach Anspruch 6, **dadurch gekennzeichnet, dass** der polymere Strukturant ein Acrylic Acid/VP Crosspolymer ist.

8. Kosmetische Reinigungszubereitungen nach wenigstens einem der vorangehenden Ansprüche, **dadurch gekennzeichnet, dass** der Gehalt an polymeren Strukturanten 0,05 bis 3,0 % Gew.-%, bevorzugt 0,1 bis 2,0 Gew.-% beträgt, bezogen auf das Gesamtgewicht der Zubereitung.

9. Kosmetische Reinigungszubereitung nach einem der vorangehenden Ansprüche, **dadurch gekennzeichnet, dass** Xanthangummi mit einem Gehalt vom 0,05 bis 0,8 Gew.-%, bevorzugt 0,1 bis 0,5 Gew.-% enthalten ist.

10. Reinigungszubereitungen nach wenigstens einem der vorangehenden Ansprüche, **dadurch gekennzeichnet, dass** die Tenside eine Kombination anionischer und nichtionische Tenside sind.

11. Reinigungszubereitungen nach wenigstens einem der vorangehenden Ansprüche, **dadurch gekennzeichnet, dass** die anionischen und/oder nichtionischen Tenside eine Alkylgruppe mit einer Kettenlänge von 10-16 C-Atomen, insbesondere von 12-14 C-Atomen aufweisen.

12. Kosmetische Reinigungszubereitungen nach wenigstens einem der vorangehenden Ansprüche, **dadurch gekennzeichnet, dass** der Tensidgehalt 0,1 bis 10 Gew.-%, bevorzugt 0,5 bis 8,0 Gew.-% beträgt, bezogen auf den Aktivgehalt und das Gesamtgewicht der Zubereitung.

13. Reinigungszubereitung nach wenigstens einem der vorangehenden Ansprüche, **dadurch gekennzeichnet, dass** als Polyole Glycerin und Propylenglycol enthalten sind.

14. Reinigungszubereitung nach wenigstens einem vorangehenden der Ansprüche **dadurch gekennzeichnet, dass** das Polyol Glycerin ist.

15. Reinigungszubereitung nach wenigstens einem vorangehenden der Ansprüche, **dadurch gekennzeichnet, dass** der Gehalt an Polyolen 15 bis 50 Gew.-%, bevorzugt 25 bis 40 Gew.-% beträgt, bezogen auf das Gesamtgewicht der Zubereitung.

16. Verwendung von polymeren Strukturanten zur Hemmung der Sedimentationsneigung von Kohlenhydratkristallen in Kohlenhydrat- und Tensid-haltigen kosmetischen Reinigungszubereitungen.

17. Verwendung nach Anspruch 16, **dadurch gekennzeichnet, dass** die Reinigungszubereitungen gemäß Anspruch 1 sind.

18. Verwendung von Polyolen, wobei als Polyole bevorzugt Propylenglykol und/oder Glycerin ausgewählt werden, mit einem Gehalt von 15 bis 50 Gew.-%, insbesondere 25 bis 40 Gew.-% zur Stabilisierung der Größe und Form von Zuckerkristallen in Reinigungszubereitungen.

19. Verwendung nach Anspruch 18, **dadurch gekennzeichnet, dass** die Reinigungszubereitungen gemäß Anspruch 1 sind.

20. Verwendung nach Anspruch 19, **dadurch gekennzeichnet, dass** der wasserlösliche Bestandteil Saccharose ist.

21. Kosmetisches, nicht-therapeutisches Verfahren zur abrasiven Hautreinigung, wobei in einem ersten Schritt Reinigungszubereitungen gemäß Anspruch 1 auf die Haut aufgetragen und verteilt werden und in einem zweiten Schritt abgespült werden.

22. Kosmetisches, nicht-therapeutisches Verfahren nach Anspruch 21, wobei der wasserlösliche Bestandteil Saccharose ist.

## Claims

1. Cleansing preparations comprising
surfactants, water and
water-soluble constituents in dissolved and crystalline form,
**characterized in that** the water-soluble constituents present are sugars, wherein a part thereof is present in the form of sugar crystals and
wherein the crystals are distributed homogeneously and in stable form in the preparation by the use of polymeric structurants and are stabilized in terms of their size and form by polyols having a content of ≥ 15% by weight, based on the total weight of the preparation, wherein the polyols are selected from the group comprising glycerol and propylene glycol.

2. Cosmetic cleansing preparations according to Claim 1, **characterized in that** the sugars are mono- and/or di- and/or oligosaccharides.

3. Cosmetic cleansing preparations according to at least one of the preceding claims, **characterized in that** the sugars are disaccharides, especially sucrose.

4. Cosmetic cleansing preparations according to at least one of the preceding claims, **characterized in that** the sugars are present at a content of 10 to 90% by weight, preferably 15 to 80% by weight, particularly preferably 20 to 70% by weight, based on the total weight of the preparation.

5. Cleansing preparation according to at least one of Claims 1 to 4, **characterized in that** the polymeric structurants are selected from the group of cellulose ethers, polyacrylates and polyacrylate copolymers.

6. Cleansing preparation according to Claim 5, **characterized in that** the polymeric structurants are polyacrylate copolymers, wherein preference is given to acrylic acid/VP crosspolymer, acrylates copolymer and acrylates crosspolymer-4.

7. Cleansing preparation according to Claim 6, **characterized in that** the polymeric structurant is an acrylic acid/VP crosspolymer.

8. Cosmetic cleansing preparations according to at least one of the preceding claims, **characterized in that** the content of polymeric structurants is 0.05 to 3.0% by weight, preferably 0.1 to 2.0% by weight, based on the total weight of the preparation.

9. Cosmetic cleansing preparation according to any of the preceding claims, **characterized in that** xanthan gum is present at a content of from 0.05 to 0.8% by weight, preferably 0.1 to 0.5% by weight.

10. Cleansing preparations according to at least one of the preceding claims, **characterized in that** the surfactants are a combination of anionic and non-ionic surfactants.

11. Cleansing preparations according to at least one of the preceding claims, **characterized in that** the anionic and/or non-ionic surfactants comprise an alkyl group having a chain length of 10-16 carbon atoms, especially 12-14 carbon atoms.

12. Cosmetic cleansing preparations according to at least one of the preceding claims, **characterized in that** the surfactant content is 0.1 to 10% by weight, preferably 0.5 to 8.0% by weight, based on the active content and the total weight of the preparation.

13. Cleansing preparation according to at least one of the preceding claims, **characterized in that** the polyols present are glycerol and propylene glycol.

14. Cleansing preparation according to at least one of the preceding claims, **characterized in that** the polyol is glycerol.

15. Cleansing preparation according to at least one of the preceding claims, **characterized in that** the content of polyols is 15 to 50% by weight, preferably 25 to 40% by weight, based on the total weight of the preparation.

16. Use of polymeric structurants for inhibiting the tendency of carbohydrate crystals to sediment in cosmetic cleansing preparations containing carbohydrate and surfactant.

17. Use according to Claim 16, **characterized in that** the cleansing preparations are according to Claim 1.

18. Use of polyols, wherein the polyols selected are preferably propylene glycol and/or glycerol at a content of 15 to 50% by weight, especially 25 to 40% by weight, for stabilizing the size and form of sugar crystals in cleansing preparations.

19. Use according to Claim 18, **characterized in that** the cleansing preparations are according to Claim 1.

20. Use according to Claim 19, **characterized in that** the water-soluble constituent is sucrose.

21. Cosmetic, non-therapeutic process for abrasive skin cleansing, wherein cleansing preparations according to Claim 1 are applied and distributed on the skin in a first step and are rinsed off in a second step.

22. Cosmetic, non-therapeutic process according to Claim 21, wherein the water-soluble constituent is sucrose.

## Revendications

1. Préparations de nettoyage, comprenant :
des tensioactifs, de l'eau et
des constituants solubles dans l'eau sous forme dissoute et cristalline,
**caractérisées en ce que** des sucres sont contenus en tant que constituants solubles dans l'eau, une partie de ceux-ci se présentant sous la forme de cristaux de sucres, et
les cristaux étant répartis de manière homogène et stable dans la préparation par l'utilisation de structurants polymères et étant stabilisés en termes de taille et de forme par des polyols en une teneur ≥ 15 % en poids, par rapport au poids total de la préparation, les polyols étant choisis dans le groupe constitué par la glycérine et le propylène glycol.

2. Préparations cosmétiques de nettoyage selon la revendication 1, **caractérisées en ce que** les sucres sont des mono- et/ou di- et/ou oligosaccharides.

3. Préparations cosmétiques de nettoyage selon au moins l'une quelconque des revendications précédentes, **caractérisées en ce que** les sucres sont des disaccharides, notamment du saccharose.

4. Préparations cosmétiques de nettoyage selon au moins l'une quelconque des revendications précédentes, **caractérisées en ce que** les sucres sont présents en une teneur de 10 à 90 % en poids, de préférence de 15 à 80 % en poids, de manière particulièrement préférée de 20 à 70 % en poids, par rapport au poids total de la préparation.

5. Préparation de nettoyage selon au moins l'une quelconque des revendications 1 à 4, **caractérisée en ce que** les structurants polymères sont choisis dans le groupe constitué par les éthers de cellulose, les polyacrylates et les copolymères de polyacrylate.

6. Préparation de nettoyage selon la revendication 5, **caractérisée en ce que** les structurants polymères sont des copolymères d'acrylate, Acrylic Acid/VP Crosspolymer, Acrylates Copolymer and Acrylates Crosspolymer-4 étant préférés.

7. Préparation de nettoyage selon la revendication 6, **caractérisée en ce que** le structurant polymère est un Acrylic Acid/VP Crosspolymer.

8. Préparations cosmétiques de nettoyage selon au moins l'une quelconque des revendications précédentes, **caractérisées en ce que** la teneur en structurants polymères est de 0,05 à 3,0 % en poids, de préférence de 0,1 à 2,0 % en poids, par rapport au poids total de la préparation.

9. Préparations cosmétiques de nettoyage selon l'une quelconque des revendications précédentes, **caractérisées en ce que** de la gomme xanthane est contenue en une teneur de 0,05 à 0,8 % en poids, de préférence de 0,1 à 0,5 % en poids.

10. Préparations de nettoyage selon au moins l'une quelconque des revendications précédentes, **caractérisées en ce que** les tensioactifs sont une combinaison de tensioactifs anioniques et non ioniques.

11. Préparations de nettoyage selon au moins l'une quelconque des revendications précédentes, **caractérisées en ce que** les tensioactifs anioniques et/ou non ioniques comprennent un groupe alkyle ayant une longueur de chaîne de 10 à 16 atomes C, notamment de 12 à 14 atomes C.

12. Préparations cosmétiques de nettoyage selon au moins l'une quelconque des revendications précédentes, **caractérisées en ce que** la teneur en tensioactif est de 0,1 à 10 % en poids, de préférence de 0,5 à 8,0 % en poids, par rapport à la teneur en agents actifs et au poids total de la préparation.

13. Préparation de nettoyage selon au moins l'une quelconque des revendications précédentes, **caractérisée en ce que** de la glycérine et du propylène glycol sont contenus en tant que polyols.

14. Préparation de nettoyage selon au moins l'une quelconque des revendications précédentes, **caractérisée en ce que** le polyol est la glycérine.

15. Préparation de nettoyage selon au moins l'une quelconque des revendications précédentes, **caractérisée en ce que** la teneur en polyols est de 15 à 50 % en poids, de préférence de 25 à 40 % en poids, par rapport au poids total de la préparation.

16. Utilisation de structurants polymères pour l'inhibition de la tendance à la sédimentation de cristaux d'hydrates de carbone dans des préparations cosmétiques de nettoyage contenant des hydrates de carbone et des tensioactifs.

17. Utilisation selon la revendication 16, **caractérisée en ce que** les préparations de nettoyage sont selon la revendication 1.

18. Utilisation de polyols, le propylène glycol et/ou la glycérine étant de préférence choisis en tant que polyols, en une teneur de 15 à 50 % en poids, notamment de 25 à 40 % en poids, pour la stabilisation de la taille et de la forme de cristaux de sucre dans des préparations de nettoyage.

19. Utilisation selon la revendication 18, **caractérisée en ce que** les préparations de nettoyage sont selon la revendication 1.

20. Utilisation selon la revendication 19, **caractérisée en ce que** le constituant soluble dans l'eau est le saccharose.

21. Procédé cosmétique non thérapeutique pour le nettoyage abrasif de la peau, dans lequel, lors d'une première étape, des préparations de nettoyage selon la revendication 1 sont appliquées et réparties sur la peau et, lors d'une deuxième étape, rincées.

22. Procédé cosmétique non thérapeutique selon la revendication 21, dans lequel le constituant soluble dans l'eau est le saccharose.
